# FASCICULE DE BREVET EUROPEEN

(11) **EP 2 929 910 B1**
(45) Date de publication et mention de la délivrance du brevet: **16.11.2016**
(21) Numéro de dépôt: 15157927.3
(22) Date de dépôt: 06.03.2015
(51) Int. Cl.: A61N 1/375, A61N 1/05, A61N 1/372, A61M 25/00

(54) **Capsule intracardiaque et son accessoire d'explantation**
Intrakardiale Kapsel und ihr Zubehör für die Explantation
Intracardiac capsule and accessory for explanting same

(30) Priorité: 08.04.2014 FR 1453132
(43) Date de publication de la demande: 14.10.2015
(73) Titulaire: Sorin CRM SAS, 92140 Clamart (FR)
(72) Inventeur: Ollivier, Jean-François, 91190 Villiers le Bacle (FR); D'hiver, Philippe, 92320 Chatillon (FR); Regnier, Willy, 91160 Longjumeau (FR)
(74) Mandataire: Dupuis-Latour, Dominique

(56) Documents cités:
- EP-A1- 2 394 695
- WO-A1-2012/082755
- US-A1- 2008 086 168
- US-A1- 2009 163 926

## Description

L'invention concerne les "dispositifs médicaux implantables actifs" tels que définis par la directive 90/385/CEE du 20 juin 1990 du Conseil des communautés européennes, notamment les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle concerne plus particulièrement, mais de façon non limitative, l'explantation de ceux de ces dispositifs qui sont pourvus à leur extrémité distale d'un organe d'ancrage de type vis hélicoïdale, prolongeant axialement le corps du dispositif et destinée à pénétrer dans le tissu cardiaque par vissage au moment de l'implantation au site choisi.

L'invention concerne notamment, mais de manière non limitative, ceux de ces dispositifs qui se présentent sous forme d'une capsule autonome destinée à être implantée dans une cavité cardiaque (ventricule ou oreillette, à droite ou à gauche). Ces capsules sont dépourvues de toute liaison mécanique à un dispositif principal implanté (tel qu'un boitier de générateur d'impulsions de stimulation) ou non implanté (périphérique externe tel que programmateur ou dispositif de *monitoring* pour le suivi à distance du patient), et sont dénommées pour cette raison "capsules *leadless",* pour les distinguer des électrodes ou des capteurs disposés à l'extrémité distale d'une sonde (*lead*) conventionnelle, qui est parcourue sur toute sa longueur par un ou plusieurs conducteurs reliant par voie galvanique l'électrode ou le capteur à un générateur connecté à une extrémité opposée, proximale, de la sonde. On notera toutefois que le caractère autonome de la capsule n'est pas intrinsèquement une caractéristique nécessaire de l'invention.

L'explantation de ces capsules autonomes est une opération particulièrement délicate car il convient, d'une part, d'arriver à saisir le corps de la capsule au moyen d'un accessoire d'explantation et, d'autre part, d'exercer sur ce corps un couple de rotation permettant de le détacher du site d'implantation où il était maintenu par la vis d'ancrage. Ce couple de dévissage doit être suffisamment important pour vaincre les résistances et adhérences résultant de la formation de tissus fibreux à l'emplacement du site d'implantation.

Dans le cas des capsules endocavitaires (c'est-à-dire des capsules à fixer à la paroi intérieure d'une cavité ventriculaire ou auriculaire, par opposition par exemple aux capsules épicardiques, fixées à la paroi extérieure du coeur), les contraintes d'explantation sont accrues du fait de la nécessité de passer par le réseau veineux périphérique d'une part pour introduire l'accessoire d'explantation, puis d'autre part pour retirer la capsule après que celle-ci a été saisie et dévissée, en assurant son retrait à travers les courbures serrées du système veineux. Ces manoeuvres doivent être exécutées de façon à la fois précise et parfaitement sécurisée.

On connait des accessoires d'explantation désignés "lassos" ou *snares,* qui sont couramment utilisés pour capturer et retirer du matériel médical tel que corps de sonde, cathéters défectueux, guides etc. hors des cavités cardiaques ou du système veineux. Ces lassos sont constitués d'un fil métallique souple terminé à son extrémité distale par une boucle déformable en métal à mémoire de forme, la boucle s'étendant à l'état libre dans un plan généralement perpendiculaire au fil métallique qui la porte. Le fil métallique est introduit dans l'orifice distal d'un cathéter, traversant celui-ci jusqu'à émerger côté proximal. La traction du fil depuis cette extrémité proximale du cathéter a pour effet, à l'autre extrémité, de tirer la boucle en la faisant progressivement entrer dans le cathéter où elle va se trouver logée.

Le cathéter est introduit dans le corps du patient, avec la boucle entièrement repliée dans la zone d'extrémité distale. La boucle est ensuite déployée hors du cathéter en repoussant le fil depuis l'extrémité proximale : du fait de la mémoire de forme du métal, la boucle reprend alors sa forme de lasso incliné par rapport à la direction du fil et du cathéter. Ce lasso peut être orienté à volonté pour capturer l'élément à extraire. Une traction exercée sur le fil permet alors de faire rentrer partiellement la boucle dans le cathéter, ce qui a pour effet d'en réduire la dimension et d'assurer ainsi le serrage de l'élément à retirer.

Ces accessoires présentent l'avantage d'avoir un faible diamètre d'introduction (de l'ordre de 2 à 6 French, 0,66 à 2 mm) tout en présentant un fort diamètre de capture (typiquement de l'ordre de 10 à 30 mm).

En revanche, dans l'application envisagée consistant à dévisser un dispositif tel qu'une capsule vissée dans une paroi, ces accessoires ne sont pas appropriés, car ils ne permettent pas de transmettre un couple significatif, alors que pour extraire une capsule vissée il est nécessaire d'exercer jusqu'à 1 N.cm sur cette capsule.

De plus, ce couple de dévissage doit être exercé sensiblement dans l'axe de la capsule. Or, avec un accessoire conventionnel de type lasso, le cathéter servant à contrôler la taille de la boucle du lasso a tendance à se placer perpendiculairement à l'élément saisi par le lasso (c'est-à-dire que l'axe de l'élément s'oriente perpendiculairement à la direction du cathéter), au mieux parallèlement à une génératrice de l'élément. Or dans une telle configuration toute action de dévissage provoquerait un vrillage des tissus autour de la vis de fixation, sans effet significatif de dévissage et avec un risque élevé de tamponnade.

Enfin, ce mode de préhension où le corps allongé d'une capsule autonome se trouverait orienté perpendiculairement à l'axe du cathéter (l'ensemble prenant donc une forme de T) serait tout à fait incompatible avec un retrait en sécurité à travers le réseau veineux.

Le WO 2012/082755 A1 décrit un accessoire d'explantation de capsule *leadless,* comprenant un ou plusieurs lassos venant saisir un organe formé en partie postérieure de la capsule, par exemple sous forme d'un bouton, ou de becs ou crochets saillants. L'organe d'accostage du cathéter comporte pour sa part un réceptacle venant s'emboiter sur la partie postérieure de la capsule après que celle-ci a été capturée par le(s) lasso(s), de manière à pouvoir transmettre le couple de dévissage nécessaire à l'explantation. Cette structure est cependant relativement complexe sur le plan mécanique, et en outre elle ne résout pas la difficulté de la manoeuvre de capture par le lasso, qui doit venir saisir des becs ou crochets de dimension très réduite.

Le US 2009/0163926 A1 décrit un cathéter à lasso destiné à l'explantation de dispositifs angéiologiques tels que des filtres insérés dans des veines, où le problème de la transmission d'un couple de dévissage relativement important ne se pose pas du tout.

Pour résoudre ces inconvénients, l'invention propose, d'une part, de réaliser un accessoire d'explantation spécifique à partir d'éléments connus mais jusqu'à présent employés dans des contextes différents et, d'autre part, d'aménager sur le corps de la capsule une rainure de capture ou organe analogue permettant de diriger la boucle du lasso vers un point situé à proximité de l'axe de la capsule.

Plus précisément, l'invention propose un ensemble comprenant, de manière en elle-même connue d'après le WO 2012/082755 A1 précité :
- une capsule autonome, comprenant un corps tubulaire pourvu à son extrémité distale d'un organe d'ancrage à vis apte à pénétrer dans un tissu d'une paroi d'un organe d'un patient ; et
- un accessoire d'explantation, comprenant un cathéter et un lasso comprenant un fil souple mobile à coulissement dans le cathéter et pourvu à son extrémité distale émergeante d'une boucle déformable, cette boucle étant apte à être serrée par introduction progressive de ses extrémités dans le cathéter sous l'effet d'une traction exercée sur le fil souple,
   le cathéter étant un cathéter armé apte à transmettre un effort de traction et un couple axial de rotation de son extrémité proximale jusqu'à son extrémité distale, et
   la capsule comportant dans sa région proximale un organe de capture apte à recevoir la boucle du lasso lors du serrage de celle-ci et à assujettir en traction et en rotation l'ensemble formé par le cathéter, la capsule et le lasso après serrage de cette boucle.

De façon caractéristique de l'invention, l'organe de capture comprend au moins une gorge de capture formée sur la région d'extrémité proximale de la capsule, cette gorge s'étendant suivant un contour curviligne orienté dans son ensemble selon un plan oblique par rapport à l'axe du corps tubulaire, et ce contour étant un contour ouvert dont les deux extrémités débouchent côté proximal au voisinage d'un point d'extrémité axiale du corps tubulaire.

Selon diverses caractéristiques subsidiaires avantageuses :
- le plan oblique suivant lequel est orienté le contour curviligne de la gorge de capture forme un angle compris entre 30° et 60° par rapport à l'axe du corps tubulaire ;
- la gorge de capture est symétrique par rapport à un plan axial longitudinal du corps tubulaire ;
- le profil de la section droite de la gorge de capture présente côté proximal un angle de contre-dépouille ;
- l'ensemble comprend deux gorges de capture symétriques diamétralement opposées par rapport à l'axe du corps tubulaire ;
- les dimensions en profondeur et en largeur de la gorge sont comprises entre 1 et 3 fois le diamètre du fil souple du lasso dans la région de la boucle ;
- le cathéter armé porte à son extrémité distale un embout spécifique avec une partie d'embout distale rigide prolongeant le cathéter au-delà de son extrémité distale, et une partie d'embout proximale montée sur le cathéter et élastiquement déformable de manière à ménager des degrés de liberté en flexion axiale pour la partie d'embout distale rigide ;
- cette partie d'embout distale rigide comprend des encoches symétriques de réception des extrémités de la boucle du lasso ;
- l'embout spécifique précité est un ressort hélicoïdal comprenant successivement une première série de spires enserrant l'extrémité distale du cathéter et formant la partie d'embout distale rigide, une deuxième série de spires s'étendant au-delà de l'extrémité distale du cathéter et formant la partie d'embout proximale, et une troisième série de spires, à l'extrémité libre du ressort, avec des spires jointives et des encoches symétriques de réception des extrémités de la boucle du lasso ;
- l'ensemble comprend, outre la capsule autonome et l'accessoire d'explantation, un cathéter téléorientable apte à recevoir le cathéter armé avec le fil du lasso introduit dedans, ce cathéter téléorientable comprenant à son extrémité distale un embout tubulaire de protection définissant un volume intérieur dimensionné de manière à pouvoir y loger la capsule après explantation.
L'invention vise également, considérés isolément, i) la capsule de cet ensemble, munie de l'organe de capture en partie proximale, et ii) l'accessoire d'explantation de cet ensemble, adapté à l'extraction d'une telle capsule.

On va maintenant décrire un exemple de mise en oeuvre de la présente invention, en référence aux dessins annexés où les mêmes références désignent d'une figure à l'autre des éléments identiques ou fonctionnellement semblables.

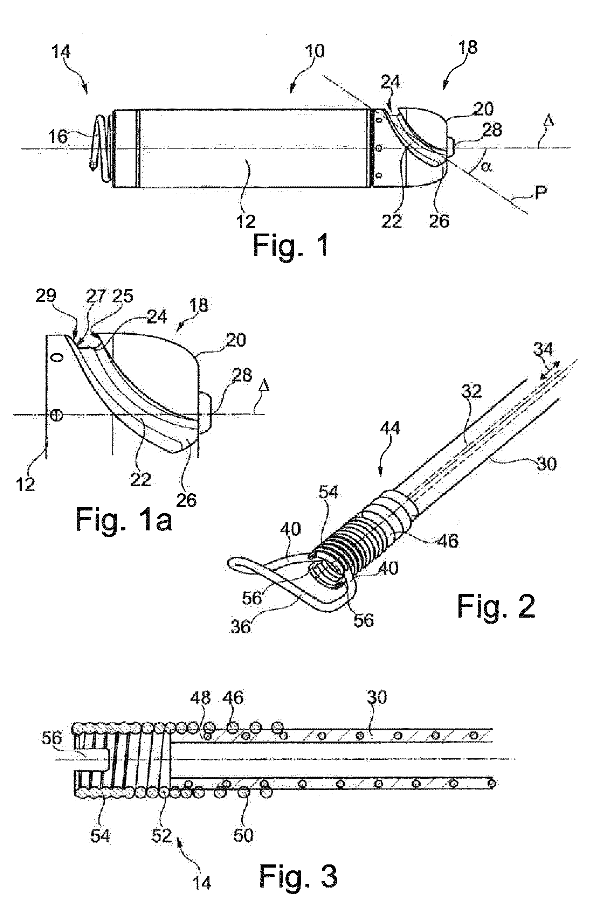

La Figure 1 est une vue latérale d'une capsule intracardiaque de type *leadless* selon un mode de réalisation de l'invention, avec une rainure de capture.
La Figure 1 a est une vue agrandie partielle de la Figure 1, montrant le détail de la région proximale de la capsule.
La Figure 2 est une vue en perspective de l'extrémité distale de l'accessoire d'explantation selon l'invention, avec un cathéter armé muni de son embout spécifique dans lequel a été introduit un lasso de capture.
La Figure 3 est une vue en coupe longitudinale de l'extrémité distale du cathéter armé de l'accessoire de la Figure 2, sans le lasso.
La Figure 4 est une vue de côté montrant la capsule de la Figure 1, avec l'accessoire d'explantation représenté dans deux positions fonctionnelles successives, respectivement en début et en fin de manoeuvre de solidarisation de l'accessoire avec la capsule.
La Figure 5 est homologue de la Figure 4, vue en perspective.
La Figure 6 est un organigramme des étapes successives d'un processus d'explantation au moyen de l'accessoire de l'invention.
La Figure 7 illustre une variante de réalisation de la capsule de la Figure 1, avec deux rainures de capture identiques diamétralement opposées.
La Figure 8 est une vue de détail, en perspective, de la variante de la Figure 7 montrant la région proximale de la capsule solidarisée à l'accessoire d'explantation.
La Figure 9 illustre un mode de réalisation où l'organe de capture est constitué d'une pluralité de crochets d'arrimage.

Sur les Figures 1, 1a, 4 et 5, on a représenté un mode de réalisation de la capsule de l'invention.

Sur ces figures, la référence 10 désigne de façon générale la capsule, réalisée sous forme d'un corps tubulaire cylindrique 12 d'axe Δ enfermant les divers circuits électroniques et d'alimentation de la capsule. Les dimensions typiques d'une telle capsule sont un diamètre de l'ordre de 6 mm environ pour une longueur d'environ 25 mm.

À son extrémité distale 14, la capsule porte une vis d'ancrage hélicoïdale 16 permettant sa fixation dans les tissus, par exemple contre une paroi d'une cavité cardiaque. Cette vis peut éventuellement être une vis active, électriquement conductrice, pour le recueil de potentiels de dépolarisation cardiaque et/ou l'application d'impulsions de stimulation.

La région proximale 18 de la capsule 10 présente de préférence une extrémité arrondie, atraumatique 20 et elle est pourvue d'un organe de capture qui sera utilisé lors de l'explantation de la capsule.

Dans le mode de réalisation illustré Figures 1, 1a, 4 et 5, l'organe de capture est constitué d'une gorge de capture 22. Cette gorge s'étend suivant un contour curviligne en forme approximative de fer à cheval, orienté dans son ensemble selon un plan P oblique par rapport à l'axe Δ du corps tubulaire, le plan P formant typiquement un angle α compris entre 30 et 60° par rapport à l'axe Δ. Le contour curviligne de la gorge 22 est symétrique par rapport à un plan axial longitudinal du corps tubulaire (plan contenant Δ). Le contour de la gorge de capture 22 est, dans l'exemple illustré, un contour unique, ouvert, avec une région centrale 24 à partir de laquelle deux bras s'étendent en direction proximale, ces bras étant orientés approximativement selon le plan P, avec les extrémités 26 de chacun de ces bras situées à proximité d'un point axial d'extrémité 28 de la capsule. Les dimensions en largeur et en profondeur de la gorge de capture 22 sont de l'ordre d'une à trois fois le diamètre du fil souple de la boucle du lasso qui sera utilisé pour l'explantation de la capsule (voir ci-après).

On notera que le profil de la section droite de la gorge de capture 22 est sensiblement constant sur l'intégralité du contour curviligne de la gorge de capture. Ce profil présente côté proximal un angle de contre-dépouille (référence 25 sur la Figure 1 a) tandis que, côté distal, il est incliné en pente douce (référence 27 sur la Figure 1a) pour se raccorder au corps tubulaire 12 (rayon de courbure référencé 29 sur la Figure 1 a).

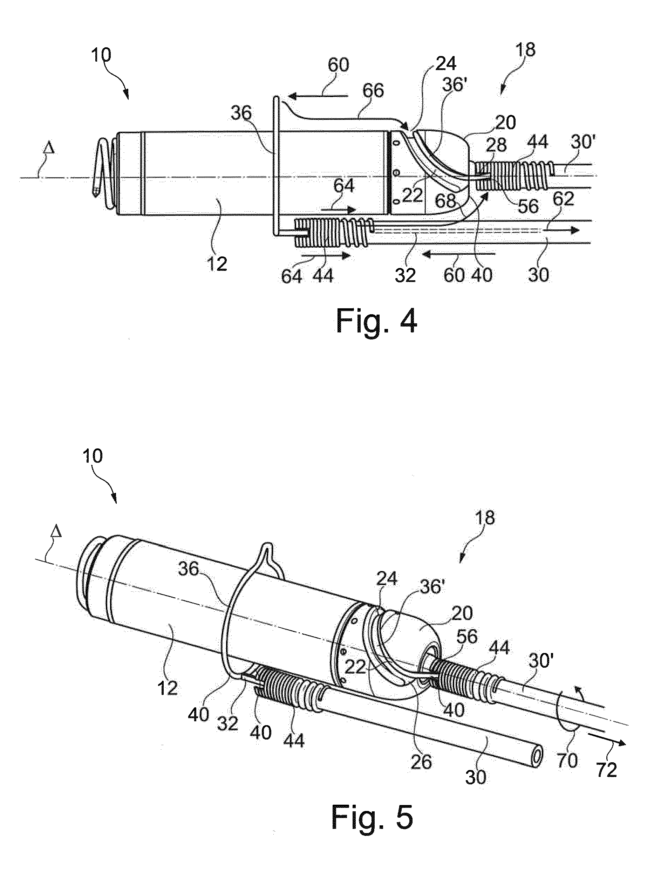

La Figure 2 illustre l'accessoire spécifique d'explantation de l'ensemble selon l'invention représenté isolément en position fermée de la boucle (c'est-à-dire correspondant à la configuration de la boucle 36' des Figures 4 et 5), la capsule ayant été masquée sur cette Figure 2.

Cet accessoire comporte un cathéter 30 qui, de façon caractéristique de l'invention, est un cathéter armé, donc apte à transmettre, sur toute sa longueur et jusqu'à son extrémité distale, aussi bien un effort de traction qu'un couple axial de rotation, tous deux exercés depuis l'extrémité proximale du cathéter. On pourra utiliser par exemple comme cathéter un modèle *JR4* de diamètre 5 French (1,66 mm) de la société Cordis, en modifiant ce cathéter pour lui adjoindre un embout spécifique 44 comme décrit plus bas en référence aux Figures 2 et 3.

La lumière interne du cathéter 30 loge un fil souple 32 mobile en translation à l'intérieur (flèche 34) et terminé à son extrémité distale par une boucle 36. Cette boucle s'étend, à l'état libre, dans un plan sensiblement perpendiculaire à la direction du fil souple 32 (comme cela est visible sur les Figures 4 et 5). À partir de cet état libre, si l'on exerce une traction dans le sens proximal sur le fil souple 32, les deux extrémités 40 de la boucle vont être progressivement tirées et introduites dans le cathéter 30 (configuration illustrée Figure 2), avec pour conséquence une diminution progressive du périmètre de la boucle 36 procurant l'effet de "lasso" recherché. La manoeuvre est bien entendu réversible, pour permettre un desserrage de la boucle et un repositionnement du lasso.

La boucle est réalisée en un alliage à mémoire de forme de type nitinol permettant au lasso de retrouver sa forme arrondie de boucle et son orientation perpendiculairement au fil souple une fois la boucle totalement émergée du cathéter.

On pourra utiliser par exemple un lasso (*snare*) du type *GN2000 Gooseneck* de la société Covidien avec un diamètre de boucle de 10 à 30 mm, dimension adaptée à la capture du corps tubulaire d'une capsule intracardiaque de diamètre 6 mm environ.

Ce lasso n'est toutefois pas utilisé avec son cathéter d'origine, qui est un cathéter souple, mais en combinaison avec un cathéter armé, comme exposé plus haut.

Sur les Figures 2 et 3, on a illustré un embout spécifique 44 dont a été pourvu le cathéter 30. Le rôle de cet embout est de permettre de réaliser avec l'ensemble cathéter armé 30 + lasso 36 la manoeuvre particulière d'explantation avec dévissage de l'élément à extraire.

Cet embout spécifique 44 comprend, essentiellement, une partie rigide située au-delà de l'extrémité distale du cathéter et reliée à cette dernière par une partie suffisamment souple, élastiquement déformable, pour procurer des degrés de liberté en pivotement (en flexion axiale) entre partie rigide et extrémité du cathéter.

Dans l'exemple de réalisation illustré Figures 2 et 3, cet embout spécifique 44 est réalisé en bobinant un ressort 46 sur l'extrémité distale 48 du cathéter 30.

La partie proximale 50 de ce ressort 44 est à spires non jointives et assure un montage serré sur l'extrémité distale 48 du cathéter, de préférence avec un collage direct additionnel du ressort sur le cathéter entre les spires. Cette partie proximale 50 est prolongée au-delà du cathéter par une partie médiane 52 à spires jointives, non soudées entre elles, de manière à former une articulation élastique de type rotule entre la partie proximale 50 et une partie distale d'extrémité 54, à spires jointives rendues rigides par soudage entre elles par laser : la partie distale 54 est alors fonctionnellement assimilable à un cylindre rigide articulé sur l'extrémité 48 du cathéter par l'intermédiaire des spires de la partie médiane 52. La partie rigide de l'extrémité distale 54 de l'embout 44 comporte en outre deux encoches 56 diamétralement opposées dont la largeur et la profondeur sont légèrement supérieures au diamètre du fil de la boucle 36 du lasso, de manière à pouvoir loger et guider les extrémités 40 de cette boucle lors du serrage de cette dernière.

On notera que l'articulation élastique de type rotule peut être réalisée par d'autres moyens que l'élément à ressort hélicoïdal que l'on vient de décrire. Ainsi, une méthode alternative consiste à usiner une pièce cylindrique à paroi pleine par découpe laser pour y former les encoches d'extrémité 56, et dans une zone intermédiaire de la paroi latérale une ou plusieurs encoches hélicoïdales qui donneront à la pièce dans cette zone l'élasticité requise en flexion axiale.

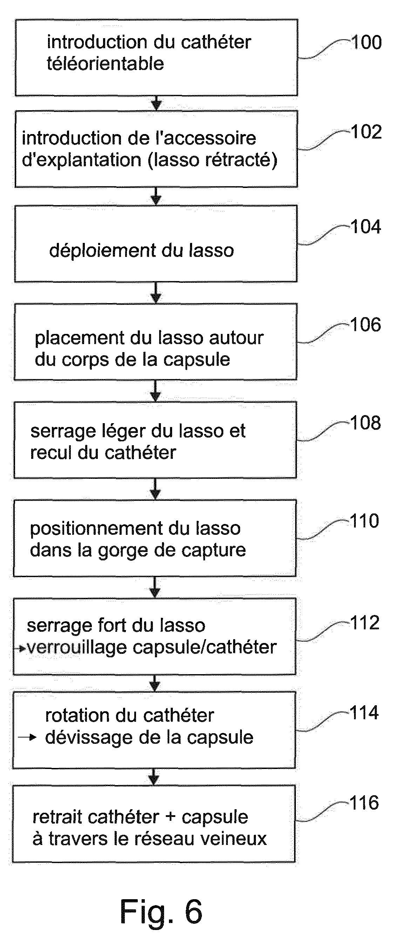

On va maintenant décrire en référence aux Figures 4 et 5 le mode opératoire de l'explantation d'une capsule intracardiaque du type que l'on vient de décrire (ou dispositif analogue). Ce mode opératoire est illustré par les étapes 100 à 116 de l'organigramme de la Figure 6.

Si cette capsule est implantée par exemple dans le ventricule droit, la première étape consiste à introduire par voie fémorale un cathéter téléorientable permettant de créer une voie d'accès jusqu'à la cavité cardiaque (Étape 100). L'accessoire d'explantation est alors introduit par cette voie d'accès, avec la boucle 36 du lasso rétractée à l'intérieur du cathéter armé 30 (Étape 102). La boucle 36 est alors déployée en repoussant le fil souple 32 (Étape 104), et le lasso est manipulé jusqu'à être placé autour du corps tubulaire 12 (Étape 106), comme illustré en 36 sur les Figures 4 et 5 (flèches 60).

Le diamètre de la boucle 36 du lasso est alors réduit en exerçant sur le fil 32 une traction dans le sens proximal (flèche 62), tout en laissant suffisamment de jeu entre la boucle 36 du lasso et le corps tubulaire 12 pour lui permettre de glisser sur ce dernier (Étape 108). Lors du serrage de la boucle, le lasso s'incline légèrement par rapport à l'axe du corps de la capsule, et le recul longitudinal progressif du cathéter (flèches 64) avec maintien de la tension de serrage du lasso permet à la boucle 36 de s'incliner légèrement par rapport à l'axe du corps tubulaire 12 et reculer (vers la droite sur les Figures 4 et 5) jusqu'à ce que la boucle vienne entrer dans la gorge de capture 22, dans la région de la partie centrale 24 de cette gorge (Étape 110).

Cette transition de la boucle 36 du centre du corps tubulaire vers la région proximale de la capsule où est située la gorge de capture 22 est illustrée par les flèches 66 et 68 sur la Figure 4.

La poursuite de ce mouvement, combiné à un maintien de la tension de serrage, permet de positionner le lasso dans l'intégralité de la rainure, comme illustré en 36' sur les Figures 4 et 5, l'extrémité du cathéter avec l'embout spécifique 44 venant alors se positionner derrière la capsule et sensiblement dans son axe, avec entre l'axe Δ du corps tubulaire 12 et l'axe du cathéter 30' un écartement ne dépassant pas 0 à 3 mm.

La manoeuvre peut être facilitée en mettant en légère rotation le cathéter armé 30 afin de placer les bras 40 du lasso dans les encoches 56 de l'embout.

Après contrôle radioscopique d'une angulation minimale entre la capsule et le cathéter armé confirmant le bon placement du lasso dans la rainure, une tension de serrage forte est alors exercée par le praticien pour verrouiller l'assemblage capsule 10 + lasso 36' + cathéter armé 30' (Étape 112).

On notera que le profil de la gorge de capture 22 participe activement au maintien de la boucle, qui est sous tension importante dans cette gorge, du fait de l'angle de contre-dépouille côté proximal (référence 25 sur la Figure 1 a). À l'inverse, le glissement de la boucle vers le fond de la rainure est aidé par le plan incliné 27 et le rayon de raccordement proximal 29 du profil de la rainure.

L'ensemble ainsi constitué est alors solidaire en traction, et également en rotation grâce au placement des bras 40 de la boucle du lasso dans les encoches 56. L'aptitude du cathéter armé 30' à transmettre un couple axial de rotation (flèche 70) est alors utilisée pour appliquer un couple de dévissage directement à la capsule, le corps du lasso (le fil souple 32) n'étant sollicité qu'en traction lors de cette étape. La capsule peut être ainsi détachée de la paroi où elle se trouvait vissée (Étape 114). L'explantation est alors achevée par traction axiale (flèche 72) sur le cathéter armé 30' et retrait de la capsule à travers le réseau veineux (Étape 116). On notera que, bien qu'il soit très résistant en traction, l'assemblage capsule 10 + lasso 36' + cathéter armé 30' reste flexible en toutes directions dans le plan longitudinal (plan contenant l'axe Δ de la capsule) du fait de l'articulation procurée par la région centrale 52 de l'embout 44. Cette propriété facilite le passage de courbures serrées et donc le trajet retour dans le réseau veineux. Un deuxième effet d'articulation dans le plan longitudinal est obtenu grâce au contact rigide sous pression, du fait de la traction sur le lasso, entre la face distale de l'embout 44 et le point axial d'extrémité 28 de la capsule.

Il est possible d'utiliser comme cathéter d'introduction (cathéter permettant de créer la voie d'accès jusqu'à la cavité cardiaque) un cathéter téléorientable comprenant à son extrémité distale un embout tubulaire de protection, comme celui décrit par exemple dans la demande française FR 13 56020 du 24.06.2013 pour *"Capsule intracardiaque et accessoire d'implantation in situ par voie fémorale".* Cet embout, situé dans une région d'approche éloignée en direction proximale du site d'implantation, recevra et logera la capsule après explantation et retrait de celle-ci jusqu'à l'embout, de sorte que le trajet final de retrait dans le réseau veineux pourra être effectué en protégeant les parois des vaisseaux de l'extrémité pointue de la vis d'ancrage 16.

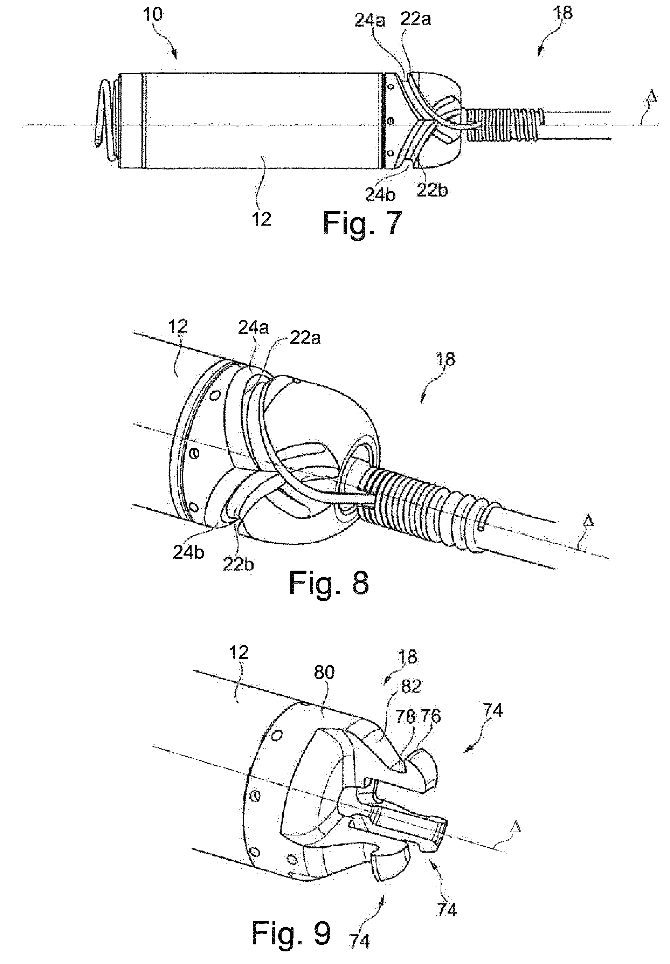

Les Figures 7 et 8 illustrent une variante de réalisation de la capsule que l'on vient de décrire, comprenant deux gorges de capture 22a, 22b diamétralement opposées. Ces deux gorges 22a, 22b définissent deux régions respectives 24a, 24b susceptibles de recevoir la boucle du lasso, ce qui facilite la capture en augmentant les chances d'accrochage par cette boucle.

La Figure 9 illustre une alternative de réalisation, où l'organe de capture est constitué non plus d'une gorge, mais de crochets d'arrimage 74, avec dans l'exemple illustré trois crochets d'arrimage identiques et répartis uniformément à 120° autour de l'axe Δ de la capsule.

Chacun de ces crochets 74 comporte un bec 76 dirigé axialement vers l'extérieur en direction radiale et définissant une zone de réception de boucle 78 plus rapprochée de l'axe Δ que l'extrémité du bec 76. Cette zone de réception 78 est reliée par une rampe de transition 82 à la région 80 prolongeant le corps tubulaire 12.

Cette configuration avec des crochets d'arrimage présente l'avantage de faciliter la prise, par la boucle du lasso, d'au moins deux crochets 74 au moment du resserrage de la boucle du lasso et du recul de celle-ci vers l'extrémité proximale 18 de la capsule après que cette dernière a été capturée par le lasso.

De façon générale, on soulignera que l'ensemble capsule/accessoire d'explantation que l'on vient de décrire présente un certain nombre d'avantages significatifs par rapport à ce qui a pu être présenté jusqu'à présent :
- système simple et fiable, dépourvu de mécanisme complexe ;
- mode opératoire proche des pratiques connues des praticiens et utilisées dans d'autres contextes ;
- la partie proximale de la capsule avec l'organe de capture (gorge de capture ou crochets d'arrimage) peut être réalisée sous forme d'un composant moulé peu onéreux ;
- le dévissage de la capsule ne nécessite pas de *redocking* pour opérer le dévissage, c'est-à-dire ne nécessite pas d'amarrage sur la capsule d'un système de gros diamètre (du même ordre que celui de la capsule), qui serait difficile à introduire jusqu'au site d'implantation et à manoeuvrer ;
- pour l'explantation, le système n'impose pas un trajet veineux de gros diamètre, comme dans le cas de l'utilisation d'une tête de *redocking.*

## Revendications

1. Un ensemble de capsule intracardiaque avec son accessoire d'explantation, cet ensemble comprenant :
- une capsule autonome (10), comprenant un corps tubulaire (12) pourvu à son extrémité distale (14) d'un organe d'ancrage à vis (16) apte à pénétrer dans un tissu d'une paroi d'un organe d'un patient ; et
- un accessoire d'explantation, comprenant :
un cathéter (30) ; et
• un lasso, comprenant un fil souple (32) mobile à coulissement (34) dans le cathéter et pourvu à son extrémité distale émergeante d'une boucle déformable (36), cette boucle étant apte à être serrée par introduction progressive de ses extrémités dans le cathéter sous l'effet d'une traction exercée sur le fil souple,
le cathéter (30) étant un cathéter armé apte à transmettre un effort de traction (72) et un couple axial de rotation (70) de son extrémité proximale jusqu'à son extrémité distale, et
la capsule comportant dans sa région proximale un organe de capture (22 ; 74) apte à recevoir la boucle du lasso lors du serrage de celle-ci et à assujettir en traction et en rotation l'ensemble formé par le cathéter, la capsule et le lasso après serrage de cette boucle,
**caractérisé en ce que** l'organe de capture comprend au moins une gorge de capture (22) formée sur la région d'extrémité proximale de la capsule, cette gorge s'étendant suivant un contour curviligne orienté dans son ensemble selon un plan oblique (P) par rapport à l'axe (Δ) du corps tubulaire (10), et ce contour étant un contour ouvert dont les deux extrémités (26) débouchent côté proximal au voisinage d'un point d'extrémité axiale (28) du corps tubulaire.

2. L'ensemble de la revendication 1, dans lequel le plan oblique (P) suivant lequel est orienté le contour curviligne de la gorge de capture forme un angle compris entre 30° et 60° par rapport à l'axe (Δ) du corps tubulaire.

3. L'ensemble de la revendication 1, dans lequel la gorge de capture (22) est symétrique par rapport à un plan axial longitudinal du corps tubulaire.

4. L'ensemble de la revendication 1, dans lequel le profil de la section droite de la gorge de capture (22) présente côté proximal un angle de contre-dépouille (25).

5. L'ensemble de la revendication 1, comprenant deux gorges de capture symétriques (22a, 22b) diamétralement opposées par rapport à l'axe (Δ) du corps tubulaire.

6. L'ensemble de la revendication 1, dans lequel les dimensions en profondeur et en largeur de la gorge (22) sont comprises entre 1 et 3 fois le diamètre du fil souple du lasso dans la région de la boucle.

7. L'ensemble de la revendication 1, dans lequel le cathéter armé (30) porte à son extrémité distale un embout spécifique (44) avec :
- une partie d'embout distale (54) rigide, prolongeant le cathéter au-delà de son extrémité distale ; et
- une partie d'embout proximale (50, 52) montée sur le cathéter et élastiquement déformable de manière à ménager des degrés de liberté en flexion axiale pour la partie d'embout distale rigide.

8. L'ensemble de la revendication 7, dans lequel la partie d'embout distale rigide comprend des encoches symétriques (56) de réception des extrémités de la boucle du lasso.

9. L'ensemble de la revendication 7, dans lequel l'embout spécifique (44) est un ressort hélicoïdal (46) comprenant successivement :
- une première série de spires (50) enserrant l'extrémité distale du cathéter et formant la partie d'embout distale rigide ;
- une deuxième série de spires (52) s'étendant au-delà de l'extrémité distale du cathéter et formant la partie d'embout proximale ; et
- une troisième série de spires (54), à l'extrémité libre du ressort, avec des spires jointives et des encoches symétriques (56) de réception des extrémités de la boucle du lasso.

10. L'ensemble de la revendication 1, dans lequel l'ensemble comprend, outre la capsule autonome et l'accessoire d'explantation :
- un cathéter téléorientable apte à recevoir le cathéter armé avec le fil du lasso introduit dedans, ce cathéter téléorientable comprenant à son extrémité distale un embout tubulaire de protection définissant un volume intérieur dimensionné de manière à pouvoir y loger la capsule après explantation.

11. Une capsule autonome (10) pour un ensemble selon l'une des revendications 1 à 10, cette capsule comprenant un corps tubulaire (12) pourvu à son extrémité distale (14) d'un organe d'ancrage à vis (16) apte à pénétrer dans un tissu d'une paroi d'un organe d'un patient,
cette capsule comportant dans sa région proximale un organe de capture (22 ; 74) apte à recevoir une boucle (36) d'un lasso avec possibilité de serrage de la boucle de manière à assujettir en traction et en rotation l'ensemble formé par la capsule et le lasso après serrage de cette boucle, **caractérisée en ce que** l'organe de capture comprend au moins une gorge de capture (22) formée sur la région d'extrémité proximale de la capsule, cette gorge s'étendant suivant un contour curviligne orienté dans son ensemble selon un plan oblique (P) par rapport à l'axe (Δ) du corps tubulaire (10), et ce contour étant un contour ouvert dont les deux extrémités (26) débouchent côté proximal au voisinage d'un point d'extrémité axiale (28) du corps tubulaire.

12. La capsule autonome de la revendication 11, dans laquelle le plan oblique (P) suivant lequel est orienté le contour curviligne de la gorge de capture forme un angle compris entre 30° et 60° par rapport à l'axe (Δ) du corps tubulaire.

13. La capsule autonome de la revendication 11, dans laquelle la gorge de capture (22) est symétrique par rapport à un plan axial longitudinal du corps tubulaire.

14. La capsule autonome de la revendication 11, dans lequel le profil de la section droite de la gorge de capture (22) présente côté proximal un angle de contre-dépouille (25).

15. La capsule autonome de la revendication 11, comprenant deux gorges de capture symétriques (22a, 22b) diamétralement opposées par rapport à l'axe (Δ) du corps tubulaire.

16. La capsule autonome de la revendication 11, dans laquelle les dimensions en profondeur et en largeur de la gorge (22) sont comprises entre 1 et 3 fois le diamètre du fil souple du lasso dans la région de la boucle.

17. Un accessoire d'explantation pour un ensemble selon l'une des revendications 1 à 10, cet accessoire comprenant :
- un cathéter (30) ; et
- un lasso, comprenant un fil souple (32) mobile à coulissement (34) dans le cathéter et pourvu à son extrémité distale émergeante d'une boucle déformable (36), cette boucle étant apte à être serrée par introduction progressive de ses extrémités dans le cathéter sous l'effet d'une traction exercée sur le fil souple,
**caractérisé en ce que** le cathéter (30) est un cathéter armé apte à transmettre un effort de traction (72) et un couple axial de rotation (70) de son extrémité proximale jusqu'à son extrémité distale.

18. L'accessoire d'explantation de la revendication 17, dans lequel le cathéter armé porte à son extrémité distale un embout spécifique (44) avec :
- une partie d'embout distale (54) rigide, prolongeant le cathéter au-delà de son extrémité distale ; et
- une partie d'embout proximale (50, 52) montée sur l'embout et élastiquement déformable de manière à ménager des degrés de liberté en flexion axiale pour la partie d'embout distale rigide.

19. L'accessoire d'explantation de la revendication 18, dans lequel la partie d'embout distale rigide comprend des encoches symétriques (56) de réception des extrémités de la boucle du lasso.

20. L'accessoire d'explantation de la revendication 18, dans lequel l'embout spécifique (44) est un ressort hélicoïdal (46) comprenant successivement :
- une première série de spires (50) enserrant l'extrémité distale du cathéter et formant la partie d'embout distale rigide ;
- une deuxième série de spires (52) s'étendant au-delà de l'extrémité distale du cathéter et formant la partie d'embout proximale ; et
- une troisième série de spires (54), à l'extrémité libre du ressort, avec des spires jointives et des encoches symétriques (56) de réception des extrémités de la boucle du lasso.

## Patentansprüche

1. Anordnung einer endokardialen Kapsel mit ihrem Entnahmezubehör, wobei diese Anordnung Folgendes umfasst:
- eine autonome Kapsel (10), die einen rohrförmigen Körper (12) aufweist, der an seinem distalen Ende (14) mit einem Schraubverankerungsorgan (16) versehen ist, das in ein Gewebe einer Wand eines Organs eines Patienten eindringen kann; und
- ein Entnahmezubehör, das Folgendes umfasst:
- einen Katheter (30); und
- ein Lasso, das einen biegsamen Draht (32) enthält, der in dem Katheter gleitend (34) beweglich ist und an seinem vorstehenden distalen Ende mit einer verformbaren Schlaufe (36) versehen ist, wobei diese Schlaufe durch allmähliches Einführen ihrer Enden in den Katheter unter der Wirkung eines auf den biegsamen Draht ausgeübten Zugs eingeklemmt werden kann,
wobei der Katheter (30) ein bewehrter Katheter ist, der eine Zugkraft (72) und ein axiales Drehmoment (70) seines proximalen Endes bis an sein distales Ende übertragen kann, und
wobei die Kapsel in ihrem proximalen Bereich ein Einfangorgan (22; 74) aufweist, das die Schlaufe des Lassos dann, wenn diese eingeklemmt wird, aufnehmen kann und die durch den Katheter, die Kapsel und das Lasso gebildete Anordnung nach dem Einklemmen dieser Schlaufe in Zug- und in Drehrichtung befestigen kann,
**dadurch gekennzeichnet, dass** das Einfangorgan wenigstens eine Einfangkehle (22) aufweist, die in dem proximalen Endbereich der Kapsel gebildet ist, wobei diese Kehle längs eines gekrümmten Umfangs, der als Ganzes in einer in Bezug auf die Achse (Δ) des rohrförmigen Körpers (10) geneigten Ebene (P) orientiert ist, verläuft, wobei dieser Umfang ein offener Umfang ist, dessen zwei Enden (26) auf proximaler Seite in die Umgebung eines axialen Endpunkts (28) des rohrförmigen Körpers münden.

2. Anordnung nach Anspruch 1, wobei die geneigte Ebene (P), längs derer der gekrümmte Umfang der Einfangkehle orientiert ist, einen Winkel im Bereich von 30° bis 60° in Bezug auf die Achse (Δ) des rohrförmigen Körpers bildet.

3. Anordnung nach Anspruch 1, wobei die Einfangkehle (22) in Bezug auf eine zu dem rohrförmigen Körper longitudinale axiale Ebene symmetrisch ist.

4. Anordnung nach Anspruch 1, wobei das Profil des Querschnitts der Einfangkehle (22) auf proximaler Seite einen Hinterschneidungswinkel (25) aufweist.

5. Anordnung nach Anspruch 1, die zwei symmetrische Einfangkehlen (22a, 22b), die in Bezug auf die Achse (Δ) des rohrförmigen Körpers diametral entgegengesetzt sind, aufweist.

6. Anordnung nach Anspruch 1, wobei die Abmessungen in Tiefen- und Breitenrichtung der Kehle (22) im Bereich des 1- bis 3-fachen Durchmessers des biegsamen Drahts des Lassos im Bereich der Schlaufe liegen.

7. Anordnung nach Anspruch 1, wobei der bewehrte Katheter (30) an seinem distalen Ende einen spezifischen Ansatz (44) trägt, mit:
- einem starren distalen Ansatzabschnitt (54), der den Katheter über ein distales Ende hinaus verlängert; und
- einem proximalen Ansatzabschnitt (50, 52), der an dem Katheter montiert ist und elastisch verformbar ist, derart, dass für den starren distalen Ansatzabschnitt Freiheitsgrade für eine axiale Biegung geschaffen werden.

8. Anordnung nach Anspruch 7, wobei der starre distale Ansatzabschnitt symmetrische Kerben (56) für die Aufnahme der Enden der Schlaufe des Lassos aufweist.

9. Anordnung nach Anspruch 7, wobei der spezifische Ansatz (44) eine Schraubenfeder (46) ist, die nacheinander Folgendes umfasst:
- eine erste Reihe von Windungen (50), die das distale Ende des Katheters einklemmen und den starren distalen Ansatzabschnitt bilden;
- eine zweite Reihe von Windungen (52), die sich über das distale Ende des Katheters hinaus erstrecken und den proximalen Ansatzabschnitt bilden; und
- eine dritte Reihe von Windungen (54) am freien Ende der Feder, die mit aneinander stoßenden Windungen und symmetrischen Kerben (56) für die Aufnahme der Enden der Schlaufe des Lassos versehen sind.

10. Anordnung nach Anspruch 1, wobei die Anordnung außer der autonomen Kapsel und dem Entnahmezubehör Folgendes umfasst:
- einen teleorientierbaren Katheter, der den bewehrten Katheter mit dem in ihn eingeführten Draht des Lassos aufnehmen kann, wobei dieser teleorientierbare Katheter an seinem distalen Ende einen rohrförmigen Schutzansatz aufweist, der ein Innenvolumen definiert, das in der Weise bemessen ist, dass die Kapsel nach ihrer Entnahme darin aufgenommen werden kann.

11. Autonome Kapsel (10) für eine Anordnung nach einem der Ansprüche 1 bis 10, wobei diese Kapsel einen rohrförmigen Körper (12) umfasst, der an seinem distalen Ende (14) mit einem Schraubeinhakorgan (16) versehen ist, das in ein Gewebe einer Wand eines Organs eines Patienten eindringen kann,
wobei diese Kapsel in ihrem proximalen Bereich ein Aufnahmeorgan (22; 74) umfasst, das eine Schlaufe (36) eines Lassos aufnehmen kann, wobei die Schlaufe in der Weise festgeklemmt werden kann, dass die durch die Kapsel und das Lasso gebildete Anordnung nach dem Festklemmen dieser Schlaufe in Zug- und in Drehrichtung befestigt werden kann,
**dadurch gekennzeichnet, dass** das Einfangorgan wenigstens eine Einfangkehle (22) aufweist, die in dem proximalen Endbereich der Kapsel gebildet ist, wobei diese Kehle längs eines gekrümmten Umfangs, der als Ganzes in einer in Bezug auf die Achse (Δ) des rohrförmigen Körpers (10) geneigten Ebene (P) orientiert ist, verläuft, wobei dieser Umfang ein offener Umfang ist, dessen zwei Enden (26) auf proximaler Seite in die Umgebung eines axialen Endpunkts (28) des rohrförmigen Körpers münden.

12. Autonome Kapsel nach Anspruch 11, wobei die geneigte Ebene (P), längs derer der gekrümmte Umfang der Einfangkehle orientiert ist, einen Winkel im Bereich von 30° bis 60° in Bezug auf die Achse (Δ) des rohrförmigen Körpers bildet.

13. Autonome Kapsel nach Anspruch 11, wobei die Einfangkehle (22) in Bezug auf eine longitudinale axiale Ebene des rohrförmigen Körpers symmetrisch ist.

14. Autonome Kapsel nach Anspruch 11, wobei das Profil des Querschnitts der Einfangkehle (22) auf proximaler Seite einen Hinterschneidungswinkel (25) aufweist.

15. Autonome Kapsel nach Anspruch 11, die zwei symmetrische Einfangkehlen (22a, 22b) aufweist, die in Bezug auf die Achse (Δ) des rohrförmigen Körpers diametral entgegengesetzt sind.

16. Autonome Kapsel nach Anspruch 11, wobei die Abmessungen in Tiefenrichtung und in Breitenrichtung der Kehle (22) im Bereich des 1- bis 3-fachen Durchmessers des biegsamen Drahts des Lassos im Bereich der Schlaufe liegen.

17. Entnahmezubehör für eine Anordnung nach einem der Ansprüche 1 bis 10, wobei dieses Zubehör Folgendes umfasst:
- einen Katheter (30); und
- ein Lasso, das einen biegsamen Draht (32) aufweist, der in dem Katheter gleitend (34) beweglich ist und an seinem vorstehenden distalen Ende mit einer verformbaren Schlaufe (36) versehen ist, wobei diese Schlaufe durch allmähliches Einführen ihrer Enden in den Katheter unter der Wirkung eines auf den biegsamen Draht ausgeübten Zugs festgeklemmt werden kann,
**dadurch gekennzeichnet, dass** der Katheter (30) ein bewehrter Katheter ist, der eine Zugkraft (72) und ein axiales Drehmoment (70) von seinem proximalen Ende bis zu seinem distalen Ende übertragen kann.

18. Entnahmezubehör nach Anspruch 17, wobei der bewehrte Katheter an seinem distalen Ende einen spezifischen Ansatz (44) trägt, mit:
- einem starren distalen Ansatzabschnitt (54), der den Katheter über sein distales Ende hinaus verlängert; und
- einem proximalen Ansatzabschnitt (50, 52), der an dem Ansatz montiert ist und in der Weise elastisch verformbar ist, dass für den starren distalen Ansatzabschnitt Freiheitsgrade für eine axiale Biegung geschaffen werden.

19. Entnahmezubehör nach Anspruch 18, wobei der starre distale Ansatzabschnitt symmetrische Kerben (56) für die Aufnahme der Enden der Schlaufe des Lassos aufweist.

20. Entnahmezubehör nach Anspruch 18, wobei der spezifische Ansatz (44) eine Schraubenfeder (46) ist, die nacheinander Folgendes umfasst:
- eine erste Reihe von Windungen (50), die das distale Ende des Katheters einklemmen und den starren distalen Ansatzabschnitt bilden;
- eine zweite Reihe von Windungen (52), die sich über das distale Ende des Katheters hinaus erstrecken und den proximalen Ansatzabschnitt bilden; und
- eine dritte Reihe von Windungen (54) an dem freien Ende der Feder, die mit aneinander stoßenden Windungen und symmetrischen Kerben (56) für die Aufnahme der Enden der Schlaufe des Lassos versehen sind.

## Claims

1. An intracardiac capsule assembly with its explantation accessory, said assembly comprising:
- an autonomous unit (10), comprising a tubular body (12) provided at its distal end (14) with a screw-anchoring member (16) adapted to penetrate a tissue of a wall of a patient's organ; and
- an explantation accessory, comprising:
. a catheter (30); and
. a lasso, comprising a flexible wire (32) slidingly mobile (34) in the catheter and provided at its distal end exiting from a deformable loop (36), this loop being adapted to be tightened by progressive introduction of its ends into the catheter under the effect of a traction exerted on the flexible wire,
the catheter (30) being a reinforced catheter adapted to transmit a traction effort (72) and an axial rotational torque (70) from its proximal end to its distal end, and
the capsule including in its proximal region a capture member (22; 74) adapted to receive the lasso loop when the latter is tightened and to fasten in traction and rotation the assembly formed by the catheter, the capsule and the lasso after tightening of this loop,
**characterized in that** the capture member comprises at least one capture groove (22) formed on the proximal end region of the capsule, this groove extending along a curvilinear contour wholly oriented according to an oblique plane (P) with respect to the axis (Δ) of the tubular body (10), and this contour being an open contour, the two ends (26) of which opening at the proximal side near an axial end point (28) of the tubular body.

2. The assembly of claim 1, wherein the oblique plane (P) according to which the curvilinear contour of the capture groove is oriented forms an angle comprised between 30° and 60° with respect to the axis (Δ) of the tubular body.

3. The assembly of claim 1, wherein the capture groove (22) is symmetrical with respect to a longitudinal axial plane of the tubular body.

4. The assembly of claim 1, wherein the profile of the cross section of the capture groove (22) has, at the proximal side, an undercut angle.

5. The assembly of claim 1, comprising two symmetrical capture grooves (22a, 22b) diametrically opposed with respect to the axis (Δ) of the tubular body.

6. The assembly of claim 1, wherein the depth and width dimensions of the groove (22) are comprised between 1 and 3 times the diameter of the flexible wire of the lasso in the region of the loop.

7. The assembly of claim 1, wherein the reinforced catheter (30) carries at its distal end a specific tip (44) with:
- a rigid distal tip part (54), extending the catheter beyond its distal end; and
- a proximal end part (50, 52) mounted on the catheter and elastically deformable so as to provide degrees of freedom in axial bending for the rigid distal tip part.

8. The assembly of claim 7, wherein the rigid distal tip part comprises symmetrical notches (56) for receiving the ends of the lasso loop.

9. The assembly of claim 7, wherein the specific tip (44) is a helical spring (46) comprising successively:
- a first series of turns (50) surrounding tightly the distal end of the catheter and forming the rigid distal tip part;
- a second series of turns (52) extending beyond the distal end of the catheter and forming the proximal end part; and
- a third series of turns (54), at the free end of the spring, with closed turns and symmetrical notches (56) for receiving the ends of the lasso loop.

10. The assembly of claim 1, wherein the assembly comprises, in addition to the autonomous capsule and the explantation accessory:
- a tele-orientable catheter adapted to receive the reinforced catheter with the lasso wire introduced therein, this tele-orientable catheter comprising at its distal end a tubular protection tip defining an internal volume dimensioned so as to be able to house the capsule after explantation.

11. A autonomous capsule (10) for an assembly according to one of claims 1 to 10, this capsule comprising a tubular body (12) provided at its distal end (14) with a screw-anchoring member (16) adapted to penetrate a tissue of a wall of a patient's organ,
the capsule including in its proximal region a capture member (22; 74) adapted to receive a lasso loop (36) with a possibility of tightening the loop so as to fasten in traction and rotation the assembly formed by the capsule and the lasso after tightening of this loop,
**characterized in that** the capture member comprises at least one capture groove (22) formed on the proximal end region of the capsule, this groove extending along a curvilinear contour wholly oriented according to an oblique plane (P) with respect to the axis (Δ) of the tubular body (10), and this contour being an open contour, the two ends (26) of which opening at the proximal side near an axial end point (28) of the tubular body.

12. The autonomous capsule of claim 11, wherein the oblique plane (P) according to which the curvilinear contour of the capture groove is oriented forms an angle comprised between 30° and 60° with respect to the axis (Δ) of the tubular body.

13. The autonomous capsule of claim 11, wherein the capture groove (22) is symmetrical with respect to a longitudinal axial plane of the tubular body.

14. The autonomous capsule of claim 11, wherein the profile of the cross section of the capture groove (22) has, at the proximal side, an undercut angle.

15. The autonomous capsule of claim 11, comprising two symmetrical capture grooves (22a, 22b) diametrically opposed with respect to the axis (Δ) of the tubular body.

16. The autonomous capsule of claim 11, wherein the depth and width dimensions of the groove (22) are comprised between 1 and 3 times the diameter of the flexible wire of the lasso in the region of the loop.

17. An explantation accessory for an assembly according to one of claims 1 to 10, this accessory comprising :
- a catheter (30); and
- a lasso, comprising a flexible wire (32) slidingly mobile (34) in the catheter and provided at its distal end exiting from a deformable loop (36), this loop being adapted to be tightened by progressive introduction of its ends into the catheter under the effect of a traction exerted on the flexible wire,
**characterized in that** the catheter (30) is a reinforced catheter adapted to transmit a traction effort (72) and an axial rotational torque (70) from its proximal end to its distal end.

18. The explantation accessory of claim 17, wherein the reinforced catheter (30) carries at its distal end a specific tip (44) with:
- a rigid distal tip part (54), extending the catheter beyond its distal end; and
- a proximal end part (50, 52) mounted on the tip and elastically deformable so as to provide degrees of freedom in axial bending for the rigid distal tip part.

19. The explantation accessory of claim 18, wherein the rigid distal tip part comprises symmetrical notches (56) for receiving the ends of the lasso loop.

20. The explantation accessory of claim 18, wherein the specific tip (44) is a helical spring (46) comprising successively:
- a first series of turns (50) surrounding tightly the distal end of the catheter and forming the rigid distal tip part;
- a second series of turns (52) extending beyond the distal end of the catheter and forming the proximal end part; and
- a third series of turns (54), at the free end of the spring, with closed turns and symmetrical notches (56) for receiving the ends of the lasso loop.
